# EUROPEAN PATENT SPECIFICATION

(11) **EP 3 274 477 B1**
(45) Date of publication and mention of the grant of the patent: **09.06.2021**
(21) Application number: 16773861.6
(22) Date of filing: 25.03.2016
(51) Int. Cl.: C12Q 1/6883

(54) **METHOD OF IDENTIFYING RISK FOR AUTISM**
VERFAHREN ZUR ERMITTLUNG DES RISIKOS FÜR AUTISMUS
MÉTHODE D'IDENTIFICATION DU RISQUE D'AUTISME

(30) Priority: 27.03.2015 US 201562139580 P
(43) Date of publication of application: 31.01.2018
(73) Proprietor: The Johns Hopkins University, Baltimore, MD 21218 (US)
(72) Inventor: FEINBERG, Andrew, P., Baltimore, MD 21218 (US); FALLIN, Daniele, M., Baltimore, MD 21218 (US)
(74) Representative: Murgitroyd & Company
(86) International application number: PCT/US2016/024314
(87) International publication number: WO 2016/160600

(56) References cited:
- WO-A1-2013/056022
- WO-A1-2015/027119
- WO-A2-2007/044780
- WO-A2-2010/120526
- US-A1- 2010 125 042
- US-A1- 2013 109 583
- US-A1- 2014 274 748
- C LADD-ACOSTA ET AL: "Common DNA methylation alterations in multiple brain regions in autism", MOLECULAR PSYCHIATRY, vol. 19, no. 8, 3 September 2013 (2013-09-03), pages 862-871, XP055488264, GB ISSN: 1359-4184, DOI: 10.1038/mp.2013.114
- FEINBERG ET AL.: 'Paternal sperm DNA methylation associated with early signs of autism risk in an autism-enriched cohort' INT. J. EPIDEMIOL. vol. 44, no. 4, 14 April 2015, pages 1199 - 1210, XP055318716
- WONG ET AL.: 'Methylomic analysis of monozygotic twins discordant for autism spectrum disorder and related behavioural traits' MOLECULAR PSYCHIATRY vol. 19, no. 4, 23 April 2013, pages 495 - 503, XP055318717

## Description

### BACKGROUND OF THE INVENTION

### FIELD OF THE INVENTION

The invention relates generally to methylation based testing and more specifically to sperm DNA methylation as a predictive marker of autism risk.

### BACKGROUND INFORMATION

Epigenetics is the study of non-sequence information of chromosome DNA during cell division and differentiation. The molecular basis of epigenetics is complex and involves modifications of the activation or inactivation of certain genes. Additionally, the chromatin proteins associated with DNA may be activated or silenced. Epigenetic changes are preserved when cells divide. Most epigenetic changes only occur within the course of one individual organism's lifetime, but some epigenetic changes are inherited from one generation to the next.

One example of an epigenetic mechanism is DNA methylation (DNAm), a covalent modification of the nucleotide cytosine. In particular, it involves the addition of methyl groups to cytosine nucleotides in the DNA, to convert cytosine to 5-methylcytosine. DNA methylation plays an important role in determining whether some genes are expressed or not. Abnormal DNA methylation is one of the mechanisms known to underlie the changes observed with aging and development of a host of disorders including many cancers, metabolic disorders and the like.

Epigenetic mechanisms such as altered DNA methylation, have been suggested to play a role in autism, beginning with the classical association of Prader-Willi syndrome, an imprinting disorder, with autistic features. Autism spectrum disorder (ASD) is a complex neurodevelopmental disorder characterized by deficits in communication and social behaviors, as well as stereotypic movements. Recent estimates from the Autism and Developmental Disabilities Monitoring (ADDM) Network report the prevalence of ASD among eight year old US children to be 1 in 68. The recurrence rate of ASD among children with an older affect sibling is estimated at 18.7% (95% CI = 13.3% to 25.5%) in the Baby Siblings Research Consortium, representing 10-fold enrichment over the general population. Neuroanatomy evidence, teratogen exposure studies, and very early life behavior differences suggest that ASD neurobiology likely begins during the *in utero* period. Intervention research demonstrates early identification and diagnosis lead to better treatment outcomes for children with ASD, thus identification of prenatal or early life ASD risk factors are critical to disease prevention or mitigation.

The etiology of ASD is incompletely characterized, and recent heritability estimates suggest approximately equal contribution from genetic and environmental risk factors. Several large genome-wide studies have identified *de novo* and inherited genetic risk factors for ASD, yet there appears to be high genetic heterogeneity and much of the overall ASD risk has not yet been explained. Similarly, evidence of prenatal toxicant risk factors for ASD is growing, but inconsistent to date. Parental characteristics may be associated with ASD risk; numerous studies have observed associations between advanced paternal age, maternal age, or both. Parental age, particularly paternal age, has been implicated in *de novo* genetic findings for ASD and has been associated with brain expression of particular genes in offspring. Other paternal risk factors might also be important, however. *In vitro* fertilization by intracytoplasmic sperm injection is associated with an increased frequency of autism that is specific for method, with a 4.6 fold increased risk after surgical sperm extraction and >9-fold if also associated with preterm birth. Consideration of combined genetic, environmental and demographic factors is likely to yield further insights into ASD etiology.

Epigenetics, defined as genomic modifications heritable during cell division other than DNA sequence *per se,* such as DNA methylation (DNAm), represents an important intersection of genetic and environmental influences that may be particularly relevant for ASD. DNAm alterations have been observed in multiple regions of post mortem brains of individuals with ASD compared to controls and between blood samples from discordant ASD twins. Epigenetic changes to the developing fetus may underscore risk of ASD, and nutritional deprivation can impact the male germline with epigenetic changes to the offspring.

WO 2010/120526 describes methods and systems for screening for and diagnosing DNA methylation associated with Autism Spectrum Disorders. C. Ladd-Acosta et al., Molecular Psychiatry, vol. 19, no. 8, 3 September 2013, pages 862-871, describes common DNA methylation alterations in multiple brain regions in autism. WO 2015/027119 describes systems and methods for determining impact of age related changes in sperm epigenome on offspring phenotype.

### SUMMARY OF THE INVENTION

In one embodiment, the invention provides a method for determining risk of autism spectrum disorder (ASD) in an offspring subject comprising analyzing DNA methylation status in a sample containing sperm from the prospective paternal parent, wherein a methylation pattern that is different from the pattern found in a sample not associated with ASD, is indicative of a risk of ASD in the offspring, wherein determining a methylation status comprises determining the methylation at differentially methylated regions (DMRs) in the DNA, and wherein the DMRs reside on SNORD115-15, SNORD115-11, SNORD115-17, and SMYD3. In a preferred aspect, the sample is a semen sample. In a preferred aspect, the subject is a human.

In another embodiment, the invention provides a method for determining whether a subject has or is at risk of having autism spectrum disorder (ASD) comprising analyzing DNA methylation status in a DNA sample of the subject, wherein a methylation pattern that is different from the pattern found in a sample not associated with ASD, is indicative of a risk of ASD in the subject, wherein determining a methylation status comprises determining the methylation at differentially methylated regions (DMRs) in the DNA, and wherein the DMRs reside on SNORD115-15, SNORD115-11, SNORD115-17 and SMYD3. In one aspect, the sample is a blood or tissue sample. In a preferred aspect, the subject is a human. In the method of the invention, risk may be assessed as a score relative to low, moderate or high risk.

In the methods of the invention the step of determining a methylation status comprises determining the methylation status of differentially methylated regions (DMRs) in the DNA, or a gene or regulatory region thereof that is associated with ASD. While not wishing to be limited, the methylation status may be performed by one or more techniques selected from the group consisting of a nucleic acid amplification, polymerase chain reaction (PCR), methylation specific PCR, bisulfite sequencing (e.g., capture or whole genome), pyrosequencing, single-strand conformation polymorphism (SSCP) analysis, restriction analysis, microarray technology, including bead microarray technology, and proteomics. Analysis of methylation status can be done by performing bead array analysis or comprehensive high-through array-based relative methylation (CHARM) analysis on a sample of labeled, digested genomic DNA, for example. The methylation status may show as hypo- or hyper-methylation.

The DMRs may be associated with genes for neurogenesis and/or neuronal development. For illustrative purposes, the DMRs are selected from the DMRs in Table 2, Table, 6, Table 7, and Table 8, herein. Described herein, are methods wherein the DMR is associated with Prader-Willi syndrome. For example, the DMR may reside on chromosome 15.

As described herein methylation status can be determined in a gene or regulatory region thereof that is associated with ASD, for example, those set forth in Table 2, Table, 6, Table 7, and Table 8, herein. In methods of the invention, the gene or regulatory region thereof includes one or more of SNORD115-15, SNORD115-11, SNORD115-17, and SMYD3. Also described herein, the gene or regulatory region thereof may optionally include MUC17, RBM19, FAM13A, GRP125, WDR1 or a combination thereof.

Described herein, the invention may provide a plurality of nucleic acid sequences that selectively hybridize to a nucleic acid sequence, such as a differentially methylated region (DMR) set forth in Table 2, Table, 6, Table 7, and Table 8, or complement thereof. In embodiments, each of the plurality of nucleic acid sequences is about 10-55 base pairs in length.

Also described herein, the invention may provide a microarray which may be used to analyze methylation status in a sample from subject having or at risk of having ASD. For example, the microarray includes one or more of the plurality of nucleic acid sequences which selectively hybridize to a differentially methylated region (DMR) set forth in Table 2, Table, 6, Table 7, and Table 8, or complement thereof.

Also described herein, is a method of determining methylation status of genomic DNA isolated from a cell. The method provide performing an assay utilizing the microarray described herein. In one embodiment, the assay is comprehensive high-throughput array for relative methylation (CHARM) analysis on a sample of labeled, digested genomic DNA isolated from the cell. Described herein, the assay may utilize a bead array format.

Also described herein, is a kit for determining whether a subject has, or is at risk of having or inheriting, ASD. The kit includes a reagent for determining methylation status of a nucleic acid sequence, wherein the nucleic acid sequence is a differentially methylated region (DMR) associated with ASD, or a gene or regulatory region thereof which is associated with ASD; and instructions for use of the reagent. , The DMR may be one or more DMRs set forth in Table 2, Table, 6, Table 7, and Table 8. In embodiments described herein, the gene or regulatory region thereof is one or more as set forth in Table 2, Table, 6, Table 7, and Table 8. In embodiments, the reagent is an oligonucleotide probe, primer, or primer pair, or combination thereof, capable of selectively hybridizing to a gene or regulatory region thereof, or DMR associated with ASD, with or without prior bisulfite treatment of the DMR Optionally the kit includes one or more detectable labels. Further, the kit may include a plurality of oligonucleotide probes, primers, or primer pairs, or combinations thereof, capable of binding to DMRs with or without prior bisulfite treatment of the DMRs. The kit may also include an oligonucleotide primer pair that hybridizes under stringent conditions to all or a portion of the DMRs only after bisulfite treatment.

### BRIEF DESCRIPTION OF THE DRAWINGS

Figures 1A-1D are graphical representations of methylation plots for the top 4 statistical DMRs identified using CHARM and 12-month score in embodiments of the present invention.
Figure 2 is a graphical representation depicting validation of CHARM AOSI DMR values using 450k data from overlapping samples (n=30).
Figure 3 is a graphical representation showing the relationship between linear regression beta coefficients of the original bump hunting model (Beta1) and the model including principal components of race from ancestry analysis of GWAS SNP data (Beta2).
Figure 4 sets forth Table 1 relating to bivariate associations of AOSI score with demographic and laboratory variables for the EARLI study population.
Figure 5 sets forth Table 2 relating to candidate differentially methylated regions (DMRs) associated with 12-month total AOSI score, an indicator of autism risk.
Figure 6 sets forth Table 3 relating to gene ontology, biological processes enriched in differentially methylated regions associated with 12-month AOSI score.
Figure 7 sets forth Table 4 relating to association between SVs and variables for CHARM samples.
Figure 8 sets forth Table 5 relating to association between SVs and variables for 450k samples.
Figure 9 sets forth Table 6 relating to the top 193 DMRs associated with autism as determined by an embodiment of the invention.
Figure 10 sets forth Table 7 relating to validation of 450k probes.
Figure 11 sets forth Table 8 relating to overlap of AOSI DMRs.

### DETAILED DESCRIPTION OF THE INVENTION

The present invention is based on the seminal discovery of the relationship of paternal sperm DNA methylation with autism risk in offspring, by examining an enriched-risk cohort of fathers of autistic children.

As described in the Examples herein, genome-wide DNAm was examined in paternal semen biosamples obtained from an ASD enriched-risk pregnancy cohort, the Early Autism Risk Longitudinal Investigation (EARLI) cohort, to estimate associations between sperm DNAm and prospective ASD development, using a 12-month ASD symptoms assessment, the Autism Observation Scale for Infants (AOSI). Methylation data from 44 sperm samples were analyzed and run on the CHARM 3.0™ array, which contains over 4 million probes (over 7 million CpG sites), including 30 samples also run on the Illumina Infinium HumanMethylation450 ™ (450k) BeadChip ™ platform (∼485,000 CpG sites). Associated regions were also examined in an independent sample of postmortem human brain ASD and control samples for which Illumina 450K™ DNA methylation data were available.

Before the present compositions and methods are described, it is to be understood that this invention is not limited to particular compositions, methods, and experimental conditions described, as such compositions, methods, and conditions may vary. It is also to be understood that the terminology used herein is for purposes of describing particular embodiments only, and is not intended to be limiting, since the scope of the present invention will be limited only in the appended claims.

As used in this specification and the appended claims, the singular forms "a", "an", and "the" include plural references unless the context clearly dictates otherwise. Thus, for example, references to "the method" includes one or more methods, and/or steps of the type described herein which will become apparent to those persons skilled in the art upon reading this disclosure and so forth.

Unless defined otherwise, all technical and scientific terms used herein have the same meaning as commonly understood by one of ordinary skill in the art to which this invention belongs.

Using region-based statistical approaches, 193 differentially methylated regions (DMRs) were identified in paternal sperm with a family-wise empirical p value (FWER) < 0.05 associated with performance on the Autism Observational Scale for Infants (AOSI) at 12 months in offspring. The DMRs clustered near genes involved in developmental processes as set forth in Table 2, Table, 6, Table 7, and Table 8, including many genes in the *SNORD* family, within the Prader-Willi syndrome gene cluster. These results were consistent among the 75 probes on the Illumina 450K™ array that cover AOSI-associated DMRs from CHARM. Further, 18 of 75 (24%) 450K array probes showed consistent differences in the cerebellums of autistic individuals compared to controls.

These data suggest that epigenetic differences in paternal sperm may contribute to autism risk in offspring, and provide evidence that directionally consistent, potentially related epigenetic mechanisms may be operating in the cerebellum of individuals with autism.

In a high-risk autism spectrum disorder (ASD) birth cohort, paternal sperm genome-wide DNA methylation patterns are associated with 12-month ASD-related phenotypes. Observations were validated in a partially independent set of fathers using a second method of DNA methylation assessment. Regions of altered sperm DNA methylation in fathers associated with infant ASD phenotypes included overlapping genes previously associated with Prader-Willi syndrome. A significant subset of sperm DMRs associated with ASD phenotypes showed directionally consistent methylation changes in postmortem cerebellar tissue of ASD patients compared to controls.

Accordingly, in one aspect, the invention provides a method for determining risk of autism spectrum disorder (ASD) in an offspring subject. The method includes a) analyzing DNA methylation status in a sample containing sperm from the prospective paternal parent; and b) determining a deviation in the methylation status of (a) as compared to a corresponding normal sample not associated with ASD, wherein a methylation pattern that deviates from the pattern found in the corresponding sample not associated with ASD, is indicative of a risk of ASD in the offspring.

In another aspect, the invention provides a method for determining whether a subject has or is at risk of having autism spectrum disorder (ASD). The method includes a) analyzing DNA methylation status in a DNA sample of the subject; and b) determining a deviation in the methylation status of (a) as compared to a corresponding normal sample not associated with ASD, wherein a methylation pattern that is different from the pattern found in the corresponding sample not associated with ASD, is indicative of a subject having or being at risk of having ASD.

Autism, one ASD, is mostly diagnosed clinically using behavioral criteria because few specific biological markers are known for diagnosing the disease. Autism is a neuropsychiatric developmental disorder characterized by impaired verbal communication, non-verbal communication, and reciprocal social interaction. It is also characterized by restricted and stereotyped patterns of interests and activities, as well as the presence of developmental abnormalities by 3 years of age. Autism-associated disorders, diseases or pathologies can comprise any metabolic, immune or systemic disorders; gastrointestinal disorders; epilepsy; congenital malformations or genetic syndromes; anxiety, depression, or AD/HD; or speech delay and motor in-coordination.

Autism spectrum disorder (ASD) is defined by impairments in verbal and non-verbal communication, social interactions, and repetitive and stereotyped behaviors. In addition to these core deficits, previous reports indicate that the prevalence of gastrointestinal symptoms ranges widely in individuals with ASD, from 9 to 91%. Macroscopic and histological observations in ASD include findings of ileo-colonic lymphoid nodular hyperplasia (LNH), enterocolitis, gastritis and esophagitis. Associated changes in intestinal inflammatory parameters include higher densities of lymphocyte populations, aberrant cytokine profiles, and deposition of immunoglobulin (IgG) and complement C1q on the basolateral enterocyte membrane. Functional disturbances include increased intestinal permeability, compromised sulphoconjugation of phenolic compounds, deficient enzymatic activity of disaccharidases, increased secretin-induced pancreaticobiliary secretion, and abnormal Clostridia taxa. Some children placed on exclusion diets or treated with the antibiotic vancomycin are reported to improve in cognitive and social function.

In various embodiments, a genome is present in a biological sample taken from a subject. The biological sample can be virtually any biological sample, particularly a sample that contains DNA from the subject. The biological sample can be a semen or tissue sample which contains about 1 to about 10,000,000, about 1000 to about 10,000,000, or about 1,000,000 to about 10,000,000 cells. However, it is possible to obtain samples that contain smaller numbers of cells, even a single cell in embodiments that utilize an amplification protocol such as PCR. The sample need not contain any intact cells, so long as it contains sufficient biological material (e.g., protein or genetic material, such as RNA or DNA) to assess methylation status of the one or more DMRs.

In some embodiments, a biological or tissue sample can be drawn from any tissue that includes cells with DNA. A biological or tissue sample may be obtained by surgery, biopsy, swab, stool, or other collection method. In some embodiments, the sample is derived from blood, plasma, serum, lymph, nerve-cell containing tissue, cerebrospinal fluid, biopsy material, tumor tissue, bone marrow, nervous tissue, skin, hair, tears, fetal material, amniocentesis material, uterine tissue, saliva, feces, or sperm. Methods for isolating PBLs from whole blood are well known in the art.

As disclosed above, the biological sample can be a blood sample. The blood sample can be obtained using methods known in the art, such as finger prick or phlebotomy. Suitably, the blood sample is approximately 0.1 to 20 ml, or alternatively approximately 1 to 15 ml with the volume of blood being approximately 10 ml.

In the present invention, the subject is typically a human but also can be any mammal, including, but not limited to, a dog, cat, rabbit, cow, bird, rat, horse, pig, or monkey.

To examine DNAm on a genome-wide scale, comprehensive high-throughput array-based relative methylation (CHARM) analysis, which is a microarray-based method agnostic to preconceptions about DNAm, including location relative to genes and CpG content is carried out. The resulting quantitative measurements of DNAm, denoted with M, are log ratios of intensities from total (Cy3) and McrBC-fractionated DNA (Cy5): positive and negative M values are quantitatively associated with methylated and unmethylated sites, respectively.

While the present invention exemplifies the CHARM assay for detection of methylation, in fact numerous methods for analyzing methylation status (hypomethylation or hypermethylation) may be utilized. In various embodiments, the determining of methylation status in the methods of the invention is performed by one or more techniques selected from the group consisting of a nucleic acid amplification, polymerase chain reaction (PCR), methylation specific PCR, bisulfite pyrosequenceing, single-strand conformation polymorphism (SSCP) analysis, restriction analysis and microarray technology. As illustrated in the Examples herein, analysis of methylation can be performed by bisulfite genomic sequencing. Bisulfite treatment modifies DNA converting unmethylated, but not methylated, cytosines to uracil. Bisulfite treatment can be carried out using the METHYLEASY™ bisulfite modification kit (Human Genetic Signatures).

In some embodiments, bisulfite pyrosequencing, which is a sequencing-based analysis of DNA methylation that quantitatively measures multiple, consecutive CpG sites individually with high accuracy and reproducibility, may be used. Exemplary primers for such analysis are set forth in in the present disclosure.

It will be recognized that depending on the site bound by the primer and the direction of extension from a primer, that the primers listed above can be used in different pairs. Furthermore, it will be recognized that additional primers can be identified within the DMRs, especially primers that allow analysis of the same methylation sites as those analyzed with primers that correspond to the primers disclosed herein.

Bisulfite treatment can be carried out using the CpG Genome DNA Modification™ kit (Intergen, Purchase, N.Y.) with the following modifications of the manufacturer's protocol: denatured genomic DNA (4 ug) can be incubated at 55 degrees C in the dark overnight in 1100 ul of freshly prepared Reagent I, with subsequent column purification with the QIAquick PCR™ purification kit (Qiagen). Purified DNA can be treated at 37 degrees C for 15 min with freshly prepared 3 M NaOH to a final concentration of 0.3 M NaOH. Then the DNA can be precipitated with ethanol and dissolved in 40 ul of 10 mM Tris (pH 8)-1 mM EDTA for nested PCR. PCR products were purified on 2% agarose gels for direct sequencing as described above. The annealing temperature was 55 degrees C. For sequencing individual clones, the PCR products can be subcloned into a TA Cloning vector (Invitrogen, Carlsbad, Calif.) according to the manufacturer's instructions, and a series of clones, such as 10-15 clones, can be selected for sequencing.

PCR products can be purified using the QIAEX II™ gel extraction kit (Qiagen) and directly sequenced with an ABI Prism 377 DNA™ sequencer using the BigDye™ Terminator Cycle Sequencing kit following the manufacturer's protocol (PE Applied Biosystems, Foster City, Calif.).

Altered methylation can be identified by identifying a detectable difference in methylation. For example, hypomethylation can be determined by identifying whether after bisulfite treatment a uracil or a cytosine is present a particular location. If uracil is present after bisulfite treatment, then the residue is unmethylated. Hypomethylation is present when there is a measurable decrease in methylation.

In an alternative embodiment, the method for analyzing methylation of the DMR can include amplification using a primer pair specific for methylated residues within a DMR In these embodiments, selective hybridization or binding of at least one of the primers is dependent on the methylation state of the target DNA sequence (Herman et al., Proc. Natl. Acad. Sci. USA, 93:9821 (1996)). For example, the amplification reaction can be preceded by bisulfite treatment, and the primers can selectively hybridize to target sequences in a manner that is dependent on bisulfite treatment. For example, one primer can selectively bind to a target sequence only when one or more base of the target sequence is altered by bisulfite treatment, thereby being specific for a methylated target sequence.

Methods using an amplification reaction, for example methods above for detecting hypomethylation or hyprmethylation of one or more DMRs, can utilize a real-time detection amplification procedure. For example, the method can utilize molecular beacon technology (Tyagi et al., Nature Biotechnology, 14: 303 (1996)) or Taqman™ technology (Holland et al., Proc. Natl. Acad. Sci. USA, 88:7276 (1991)).

Also methyl light (Trinh et al., Methods 25(4):456-62 (2001)), Methyl Heavy (Epigenomics, Berlin, Germany), or SNuPE (single nucleotide primer extension) (see e.g., Watson et al., Genet Res. 75(3):269-74 (2000)) Can be used in the methods of the present invention related to identifying altered methylation of DMRs.

Other methods are known in the art for determining methylation status of a DMR, including, but not limited to, array-based methylation analysis and Southern blot analysis. Additionally, as mentioned above, methyl light, methyl heavy, and array-based methylation analysis can be performed, by using bisulfate treated DNA that is then PCR-amplified, against microarrays of oligonucleotide target sequences with the various forms corresponding to unmethylated and methylated DNA.

As used herein, the term "selective hybridization" or "selectively hybridize" refers to hybridization under moderately stringent or highly stringent physiological conditions, which can distinguish related nucleotide sequences from unrelated nucleotide sequences.

As known in the art, in nucleic acid hybridization reactions, the conditions used to achieve a particular level of stringency will vary, depending on the nature of the nucleic acids being hybridized. For example, the length, degree of complementarity, nucleotide sequence composition (for example, relative GC:AT content), and nucleic acid type, for example, whether the oligonucleotide or the target nucleic acid sequence is DNA or RNA, can be considered in selecting hybridization conditions. An additional consideration is whether one of the nucleic acids is immobilized, for example, on a filter. Methods for selecting appropriate stringency conditions can be determined empirically or estimated using various formulas, and are well known in the art *(see, e.g.,* Sambrook et al., supra, 1989).

An example of progressively higher stringency conditions is as follows: 2X SSC/0.1% SDS at about room temperature (hybridization conditions); 0.2X SSC/0.1% SDS at about room temperature (low stringency conditions); 0.2X SSC/0.1% SDS at about 42°C (moderate stringency conditions); and 0.1X SSC at about 68°C (high stringency conditions). Washing can be carried out using only one of these conditions, for example, high stringency conditions, or each of the conditions can be used, for example, for 10 to 15 minutes each, in the order listed above, repeating any or all of the steps listed.

The degree of methylation in the DNA associated with the DMRs being assessed, may be measured by fluorescent *in situ* hybridization (FISH) by means of probes which identify and differentiate between genomic DNAs, associated with the DMRs being assessed, which exhibit different degrees of DNA methylation. FISH is described, for example, in de Capoa et al. (Cytometry. 31:85-92, 1998). In this case, the biological sample will typically be any which contains sufficient whole cells or nuclei to perform short term culture. Usually, the sample will be a sample that contains 10 to 10,000, or, for example, 100 to 10,000, whole cells.

The term "nucleic acid molecule" is used broadly herein to mean a sequence of deoxyribonucleotides or ribonucleotides that are linked together by a phosphodiester bond. As such, the term "nucleic acid molecule" is meant to include DNA and RNA, which can be single stranded or double stranded, as well as DNA/RNA hybrids. Furthermore, the term "nucleic acid molecule" as used herein includes naturally occurring nucleic acid molecules, which can be isolated from a cell, as well as synthetic molecules, which can be prepared, for example, by methods of chemical synthesis or by enzymatic methods such as by the polymerase chain reaction (PCR), and, in various embodiments, can contain nucleotide analogs or a backbone bond other than a phosphodiester bond.

The terms "polynucleotide" and "oligonucleotide" also are used herein to refer to nucleic acid molecules. Although no specific distinction from each other or from "nucleic acid molecule" is intended by the use of these terms, the term "polynucleotide" is used generally in reference to a nucleic acid molecule that encodes a polypeptide, or a peptide portion thereof, whereas the term "oligonucleotide" is used generally in reference to a nucleotide sequence useful as a probe, a PCR primer, an antisense molecule, or the like. Of course, it will be recognized that an "oligonucleotide" also can encode a peptide. As such, the different terms are used primarily for convenience of discussion.

A polynucleotide or oligonucleotide comprising naturally occurring nucleotides and phosphodiester bonds can be chemically synthesized or can be produced using recombinant DNA methods, using an appropriate polynucleotide as a template. In comparison, a polynucleotide comprising nucleotide analogs or covalent bonds other than phosphodiester bonds generally will be chemically synthesized, although an enzyme such as T7 polymerase can incorporate certain types of nucleotide analogs into a polynucleotide and, therefore, can be used to produce such a polynucleotide recombinantly from an appropriate template.

In various aspects of the invention, methylation status is converted to an M value. As used herein an M value, can be a log ratio of intensities from total (Cy3) and McrBC-fractionated DNA (Cy5): positive and negative M values are quantitatively associated with methylated and unmethylated sites, respectively.

In various aspects of the invention DMR may be hypermethylated or hypomethylated. Hypomethylation of a DMR is present when there is a measurable decrease in methylation of the DMR. In some embodiments, a DMR can be determined to be hypomethylated when less than 50% of the methylation sites analyzed are not methylated. Hypermethylation of a DMR is present when there is a measurable increase in methylation of the DMR. In some embodiments, a DMR can be determined to be hypermethylated when more than 50% of the methylation sites analyzed are methylated. Methods for determining methylation states are provided herein and are known in the art. In some embodiments methylation status is converted to an M value. As used herein an M value, can be a log ratio of intensities from total (Cy3) and McrBC-fractionated DNA (Cy5): positive and negative M values are quantitatively associated with methylated and unmethylated sites, respectively. M values are calculated as described in the Examples. In some embodiments, M values which range from -0.5 to 0.5 represent unmethylated sites as defined by the control probes, and values from 0.5 to 1.5 represent baseline levels of methylation.

In one embodiment of the invention, methylation density is determined for a region of nucleic acid, such as a DMR. Density may be used as an indication of production of an iPS cell, for example. A density of about 0.2 to 0.7, about 0.3 to 0.7 , 0.3 to 0.6 or 0.3 to 0.4, or 0.3, may be indicative of ASD (the calculated DNA methylation density is the number of methylated CpGs divided by the total number of CpGs sequenced for each sample). Methods for determining methylation density are well known in the art. For example, a method for determining methylation density of target CpG islands has been established by Luo et al. (Analytical Biochemistry, Vol. 387:2 2009, pp. 143-149). In the method, DNA microarray was prepared by spotting a set of PCR products amplified from bisulfite-converted sample DNAs. This method not only allows the quantitative analysis of
regional methylation density of a set of given genes but also could provide information of methylation density for a large amount of clinical samples as well as use in the methods of the invention regarding iPS cell generation and detection. Other methods are well known in the art *(e.g.,* Holemon et al., BioTechniques, 43:5, 2007, pp. 683-693).

Also described herein, are kits that are useful for carrying out the methods of the present invention. The components contained in the kit depend on a number of factors, including: the particular analytical technique used to detect methylation or measure the degree of methylation or a change in methylation, and the one or more DMRs or genes being assayed for methylation status.

Accordingly, disclosed is a kit for determining a methylation status of one or more DMRs. In some embodiments, the one or more DMRs are selected from one or more of the sequences as set forth in Table 2, Table, 6, Table 7, and Table 8, or DMRs associated with Prader-Willi Syndrome, e.g., on chromosome 15. The kit includes an oligonucleotide probe, primer, or primer pair, or combination thereof for carrying out a method for detecting methylation status, as discussed above. For example, the probe, primer, or primer pair, can be capable of selectively hybridizing to the DMR either with or without prior bisulfite treatment of the DMR. The kit can further include one or more detectable labels.

The kit can also include a plurality of oligonucleotide probes, primers, or primer pairs, or combinations thereof, capable of selectively hybridizing to the DMR with or without prior bisulfite treatment of the DMR. The kit can include an oligonucleotide primer pair that hybridizes under stringent conditions to all or a portion of the DMR only after bisulfite treatment. In one aspect, the kit can provide reagents for bisulfite sequencing or pyrosequencing. The kit can include instructions on using kit components to identify, for example, the presence of autism or increased risk for developing autism.

In a related embodiment, the kit may further include computer executable code and instructions for performing statistical analysis.

Also described herein are a plurality of nucleic acid sequences that selectively hybridize to a nucleic acid sequence, such as a differentially methylated region (DMR) set forth in Table 2, Table, 6, Table 7, and Table 8, or complements thereof.

Also described herein are a plurality of nucleic acid sequences that selectively hybridize to a nucleic acid sequence including a gene or promoter region thereof as set forth in Table 2, Table, 6, Table 7, and Table 8, or complements thereof. In some embodiments, the plurality of nucleic acid sequences selectively hybridize to one or more of the following genes or regulatory regions thereof: SNORD115-15, SNORD115-11, SNORD115-17, SMYD3, MUC17, RBM19, FAM13A, GRP125, WDR1 or a combination thereof, or complements thereof.

A microarray which may be used to analyze methylation status in a sample from subject having or at risk of having ASD is disclosed herein. In one embodiment the microarray includes one or more of the plurality of nucleic acid sequences which selectively hybridize to a differentially methylated region (DMR) set forth in Table 2, Table, 6, Table 7, and Table 8, or complements thereof, or to one or more of the following genes or regulatory regions thereof: SNORD115-15, SNORD115-11, SNORD115-17, SMYD3, MUC17, RBM19, FAM13A, GRP125, WDR1 or a combination thereof, or complements thereof. One skilled in the art would appreciate the many techniques that are well known for attaching nucleic acids on a substrate that may be utilized along with the various types of substrates and configurations.

The microarray described herein may be included in the kit described herein optionally along with reagents for performing an array based assay.

Polynucleotides or nucleic acid sequences, such as oligonucleotides, primers, probes, and the like may be of any suitable length. For example, one of skill in the art would understand what lengths are suitable for nucleic acid sequences to be used in an array or kit as disclosed herein. Such molecules are typically from about 5 to 100, 5 to 50, 5 to 45, 5 to 40, 5 to 35, 5 to 30, 5 to 25, 5 to 20, or 10 to 20 nucleotides in length. For example the molecule may be about 5, 10, 15, 16, 17, 18, 19, 20, 21, 22, 23, 24, 25, 26, 27, 28, 29, 30, 31, 32, 33, 34, 35, 40, 45 or 50 nucleotides in length. Such polynucleotides may include from at least about 15 to more than about 120 nucleotides, including at least about 16 nucleotides, at least about 17 nucleotides, at least about 18 nucleotides, at least about 19 nucleotides, at least about 20 nucleotides, at least about 21 nucleotides, at least about 22 nucleotides, at least about 23 nucleotides, at least about 24 nucleotides, at least about 25 nucleotides, at least about 26 nucleotides, at least about 27 nucleotides, at least about 28 nucleotides, at least about 29 nucleotides, at least about 30 nucleotides, at least about 35 nucleotides, at least about 40 nucleotides, at least about 45 nucleotides, at least about 50 nucleotides, at least about 55 nucleotides, at least about 60 nucleotides, at least about 65 nucleotides, at least about 70 nucleotides, at least about 75 nucleotides, at least about 80 nucleotides, at least about 85 nucleotides, at least about 90 nucleotides, at least about 95 nucleotides, at least about 100 nucleotides, at least about 110 nucleotides, at least about 120 nucleotides or greater than 120 nucleotides.

The following example is provided to further illustrate the advantages and features of the present invention, but are not intended to limit the scope of the invention. While they are typical of those that might be used, other procedures, methodologies, or techniques known to those skilled in the art may alternatively be used.

### EXAMPLE 1

### PATERNAL SPERM DNA METHYLATION ANALYSTS

This example details a study in which genome-wide DNAm was examined in paternal semen biosamples obtained from an ASD enriched-risk pregnancy cohort, the Early Autism Risk Longitudinal Investigation (EARLI) cohort, to estimate associations between sperm DNAm and prospective ASD development, using a 12-month ASD symptoms assessment, the Autism Observation Scale for Infants (AOSI). Methylation data from 44 sperm samples were analyzed on the CHARM 3.0 array, which contains over 4 million probes (over 7 million CpG sites), including 30 samples also run on the Illumina Infinium HumanMethylation450™ (450k) BeadChip ™ platform (∼485,000 CpG sites). Associated regions were also examined in an independent sample of postmortem human brain ASD and control samples for which Illumina 450K™ DNA methylation data were available.

### EXPERIMENTAL MATERIALS AND METHODS

### Study sample

Semen samples (n=44) were obtained from fathers of families enrolled in the Early Autism Risk Longitudinal Investigation (EARLI), an enriched autism risk pregnancy cohort that focuses on the prenatal and early life periods of newborns who have biological siblings already diagnosed with an ASD. For diseases such as ASD that demonstrate familial aggregation, disease-specific enriched-familial risk cohort studies represent an attractive alternative to large birth cohorts. In enriched-risk studies, subjects are oversampled for increased familial risk, increasing power to detect associations between risk factors and disease. The EARLI study is comprised of four data collection sites (Drexel University, Johns Hopkins University, University of California, Davis, and Kaiser Permanente Division of Research) and includes prospective collection of biological samples from multiple family members along with environmental, questionnaire, interview, and clinical assessment data collected at multiple time points throughout the pregnancy and through age 3 of the newborn sibling. The EARLI study was reviewed and approved by Human Subjects Institutional Review Boards (IRBs) from each of the four study sites.

The samples analyzed within this project were collected from biological fathers of infant siblings using a home-collection semen kit distributed at the time of enrollment, typically in first or second trimester of pregnancy. Semen collection kits with instructions were mailed to the father of the current pregnancy prior to the first prenatal visit. The goal was to collect the semen sample as close to the time of the family's enrollment in EARLI as possible, but samples were accepted any time during the study. Paternal demographic information was collected via the EARLI paternal interview (or, if unavailable, from the maternal interview). Paternal age for each biosample was calculated by subtracting the father's date of birth from the date of sample collection.

A total of 44 sperm DNA samples were available for CHARM DNA methylation (DNAm) analysis, and an overlapping validation set of 30 samples with DNAm assessment via the Illumina 450k ™ platform (see methods below) was available. All 44 individuals had 12-month developmental assessment data from the child available.

### Phenotype assessment

EARLI siblings participated in extensive neurodevelopmental phenotyping at regular intervals during development including clinician assessment and parent report. The 12-month clinical assessment included the Autism Observation Scale for Infants (AOSI) tool, an 18-item clinician-administered, semi-structured, play-based observation that is targeted for high-risk infants. It assesses eye contact, visual tracking, imitation, atypical sensory behavior, and social babbling (among other behaviors), many of which have been associated with later ASD. AOSI scores at 12-months have been shown to predict Autism Diagnostic Observation Schedule (ADOS) classification at 24- (9) and 36-months, and are thus used in this analysis as an early, quantitative, indicator of ASD related phenotype in at-risk siblings.

### Sample processing and DNA extraction

Semen samples were frozen upon collection as per the EARLI collection kit instructions and then shipped (either same- or next-day) directly to the Johns Hopkins Biosample Repository (JHBR) for storage (-80°C) until processing. Genomic DNA for 44 samples used in CHARM analyses and for 26 of the samples used in 450K analyses was extracted from semen samples using a QIAgen QIAsymphony ™ automated workstation with the Blood 1000 ™ protocol of the DSP DNA Midi ™ kit (Cat. No. 937255, Qiagen, Valencia, CA) as per manufacturer's instructions. For the additional 4 samples measured via 450K analyses, DNA was extracted at the Johns Hopkins School of Medicine Center for Epigenetics using a version of the Epicentre MasterPure DNA Purification Kit™ (Cat. No. MCD85201).

### CHARM DNA methylation measurement

Genome-wide sperm DNA methylation was measured using the Comprehensive High-throughput Arrays for Relative Methylation assay (CHARM) (Ladd-Acosta et al., Comprehensive high-throughput arrays for relative methylation (CHARM). Current protocols in human genetics / editorial board, Jonathan L Haines et al. 2010 Apr; Chapter 20: Unit 20 1 1-19.). Genomic sperm DNA (4µg) was sheared on a HydroShear Plus™ (DigiLab, Marlborough, MA), digested with McrBC, gel-purified, labeled, and hybridized to arrays as described (Ladd-Acosta et al., supra). The CHARM 3.0™ array (Roche NimbleGen™, Madison, WI) includes over 4 million probes and covers over 7 million CpGs arranged into probe groups (consecutive probes are within 300 bp of each other). These arrays include probes covering all annotated and non-annotated promoters and microRNA sites in addition to features present in the original CHARM method (Ladd-Acosta et al., supra).

### Illumina 450k™ DNA methylation measurement

DNAm for 30 overlapping paternal semen samples was measured via the Illumina Infinium HumanMethylation450 BeadChip ™ assay (Illumina, San Diego, CA). Genomic DNA (1/µg) was processed by the Johns Hopkins University SNP Center using the automated Infinium™ workflow. Technical control samples, including technical replicates of liver and placenta, and spike-in samples (here, 0%, 50%, and 100% methylated commercial DNA) were repeated across plates to ensure consistent high quality data. Note these samples were not included in any subsequent analyses of semen.

### Data pre-processing

CHARM raw data were pre-processed using the CHARM Package™ (v.2.8.0) in R™ (version 3.0.3). Briefly, probe-level percentage DNAm estimates were obtained by first removing background signal using anti-genomic background probes, followed by normalization using control probes (loess for within arrays, followed by quantile between arrays). After normalization, we excluded background, control and repetitive probe groups, yielding 3,811,046 total probes per array for each of the 44 discovery samples. Surrogate variable analysis (SVA) was then performed on these percentage methylation estimates to estimate latent factors influencing DNAm levels which typically represent "batch" effects. The number of surrogate variables (SVs) to include in the statistical models were estimated using the Buja and Eyuboglu ("be") algorithm, which identifies how many latent variables are present in the data. Then the SVA algorithm estimates these SVs which are adjusted for as confounders in downstream differential methylation bump hunting analysis.

Validation 450k raw data were pre-processed using the Minfi™ Package (v.1.10.1) in R™ (version 3.1.0). Data were stratified quantile normalized and SVA was performed on the percentage methylation estimates (beta scale) to adjust for potential batch effects.

### Age and developmental assessment associations

Differences in AOSI score across demographic and technical/laboratory variables were compared using F-tests. Bivariate associations between 12-month AOSI scores and ordinal variables were calculated using linear model trend tests. Similar tests were also performed to assess the degree of association between SVs and demographic/laboratory variables. Spearman rank correlations were estimated between paternal age and offspring 12-month AOSI scores.

### Regional DNAm discovery

Regions of CHARM DNAm differences by infant AOSI score were identified using the "bump hunting" approach previously developed for the CHARM platform (Jaffe et al., 2012 Feb;41(1):200-9), adjusting for estimated surrogate variables. The statistical model treated AOSI as the outcome of interest, and adjusted for paternal age and 10 surrogate variables (described above) as confounders. This model is applied to every high-quality probe on the microarray to identify the adjusted linear effect of AOSI on DNA methylation levels. Regions of differential methylation were identified by smoothing the linear effects of AOSI on DNA methylation, and then thresholding these smoothed statistics across all probes using the 99.9^{th} percentile as a cutoff, as previously described (Jaffe et al., supra). P-values for each DMR were calculated from a genome-wide empirical distribution of null statistics generated using a linear model bootstrapping approach across 10,000 permutations as described (Jaffe et al., supra). DMRs with a genome-wide family-wise empirical p value (FWER) < 0.05 were identified. Note that the DMRs are ranked prior to the permutation procedure, which is used to identify the threshold that controls for the target genome-wide FWER. Mean methylation values across the DMR for each sample were also plotted by AOSI score and Spearman rank correlation coefficients estimated.

Post-hoc sensitivity analyses was performed to assess the potential influence of skewed or extreme AOSI scores and the influence of race on identified DMRs. First, to account for right-skewed AOSI scores, a natural log(AOSI+1) transformation was used in regressions against the average methylation per DMR, for each DMR with p < 0.05. A dichotomous outcome was also considered comparing the highest quartile of AOSI scores (>10) to the lowest three quartiles. These provide comparisons of effect size magnitude and direction between the discovery DMRs and these alternative forms of AOSI modeling. Bump hunting models were also reanalyzed adjusting for principal components of race (PC1 and PC2 from ancestry analysis of GWAS SNP data) and beta coefficients compared between this race-adjusted model and the primary discovery bump hunting results.

### Gene enrichment analyses

Enrichment of genes was tested for within 10kb of DMRs with FWER < 0.05 based on Gene Ontology™ (Biological Processes database) using the hypergeometric test restricted to gene sets with at least 4 members. The GOstats R Bioconductor™ package was used to compare genes mapped to top DMRs (FWER < 0.05) to all genes on the CHARM array that also have an Entrez ID as background.

### Cross-platform validation

DMRs discovered via CHARM for AOSI score were attempted to be validated using overlapping genomic coverage on the 450k array in a partially overlapping set of paternal samples. Within these subsets of genomic sites, linear regression was first used to test the relationship between single-site 450k DNAm and AOSI score. Statistical models were adjusted for surrogate variables estimated from the data of only overlapping samples (n=30 samples with 7 SVs for AOSI score). Spearman correlation tests were used to calculate the correlation between effect estimates from CHARM and 450k.

### Comparison with autism brain data

Publicly available Illumina 450k™ data from postmortem human brain tissue was downloaded on 40 samples (19 ASD and 21 controls) across 3 brain regions (frontal cortex, temporal cortex, and cerebellum) from GEO dataset: GSE53162 (25). Illumina 450k™ data were normalized as above with the FlowSorted.DLPFC.450k Bioconductor™ dataset to estimate cellular composition in each sample. Mean differences and resulting t-statistics and p-values were calculated within each brain region comparing cases to controls using the limma Bioconductor™ package, and considered probes with differential methylation with p < 0.05 marginally significant for the cross-tissue comparison analysis.

### RESULTS

### Study sample characteristics

Infant sibling performance on the 12-month AOSI for children of the fathers examined in the CHARM discovery sample ranged from 0 to 18. Fathers were predominantly white and non-Hispanic (73%) and their ages ranged between 27 to 51 years (Table 1). Sibling AOSI scores were not associated with paternal age (p= -0.04), education level or family income, though higher AOSI scores were observed at the Drexel study site.

### Methylation data quality assessment

Total AOSI did not vary by CHARM DNA shearing date (P value=0.77), HydroShear™ machine (P value=0.9) CHARM gel (P value=0.65), sample location in CHARM gel (P value=0.71), or CHARM hybridization date (P value=0.39). Table 4 shows the degree of association between CHARM variables to each of the estimated surrogate variables.

Illumina 450k™ between-plate correlation Pearson coefficients of unnormalized DNAm between technical control replicates ranged from 0.848 to 0.996 with a mean (SD) of 0.963 (0.041). DNA samples were hybridized to *Illumina* arrays across 3 dates and AOSI score did vary by hybridization date (P value=0.06). Table 5 shows the degree of association between 450k variables to each of the estimated surrogate variables.

### DNAm and AOSI score

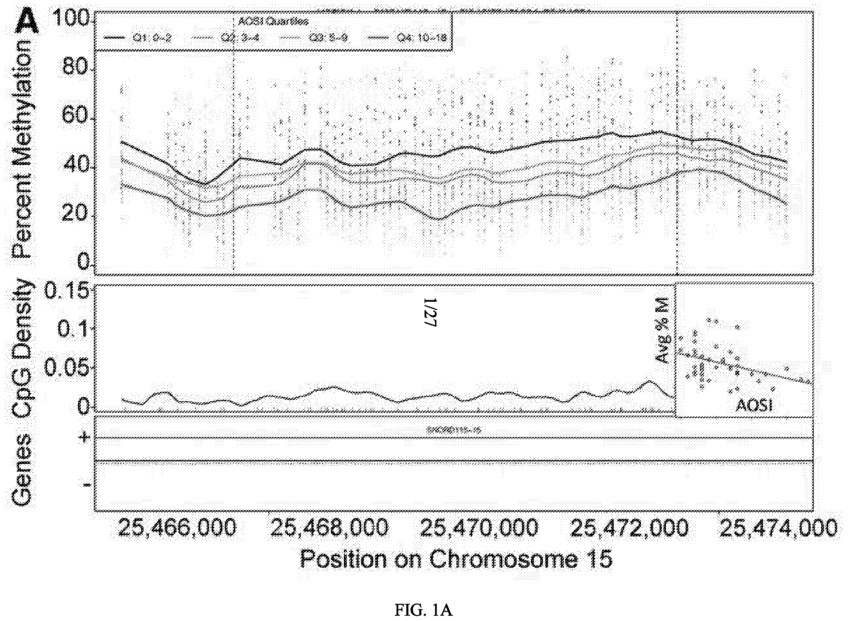

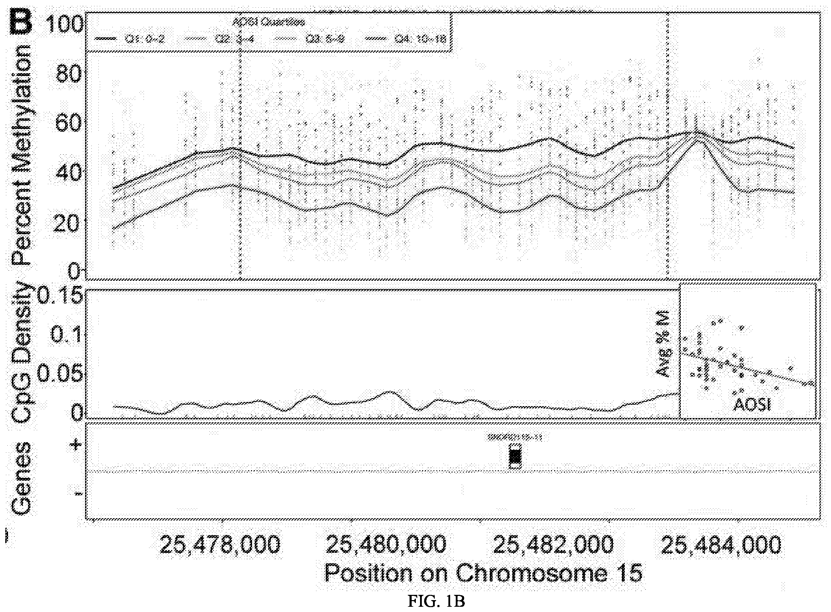

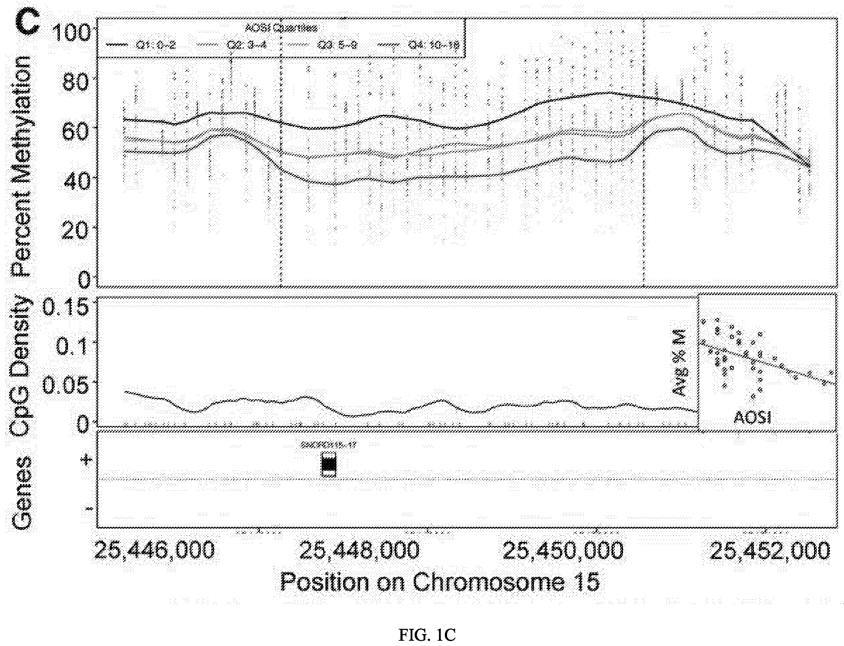

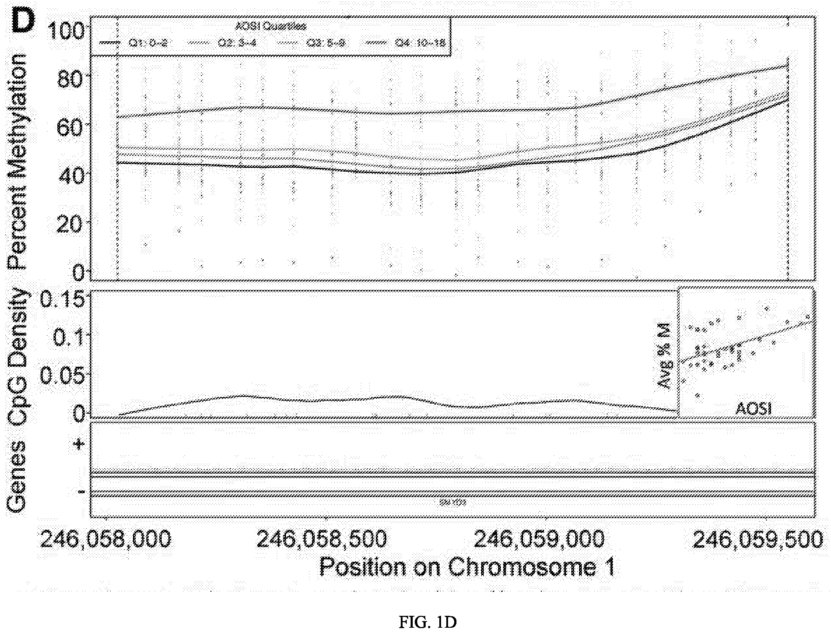

2605 candidate DMRs were identified using our "bump hunting" method in paternal sperm associated with child AOSI score at 12 months, and after permutation analysis, the top 193 DMRs had genome-wide p < 0.05. The top 10 ranked DMRs are shown in Table 2, and Table 6 includes the complete list of 193 DMRs. Methylation plots by AOSI score for all 193 DMRs are publicly available on the World Wide Web at URL ije.oxfordjournals.org/content/44/4/1199.full. The top four regions where DNAm associates linearly (genome-wide p=0.001) with AOSI score were highlighted in Figure 1, with the average DNAm across the DMR shown in each inset. Figure 1a shows hypomethylation with increased AOSI score on chromosome 15 overlapping *SNORD115-15* where the highest quartile of AOSI scores (≥10) corresponded to 27.4% average sperm methylation compared with 46.9% average methylation for the lowest quartile (< 3). Similarly, Figures 1b and 1c show hypomethylation with increased AOSI scores on chromosome 15 covering *SNORD115-11* and *SNORD115-17,* respectively, with differences in regional average methylation between the highest and lowest AOSI quartiles of 19.7% and 22.6%, respectively. Figure 1d illustrates hypermethylation with increased AOSI scores on chromosome 1 overlapping *SMYD3,* with 68.9% average methylation for the highest AOSI score quartile compared to 47.3% average methylation of the lowest quartile.

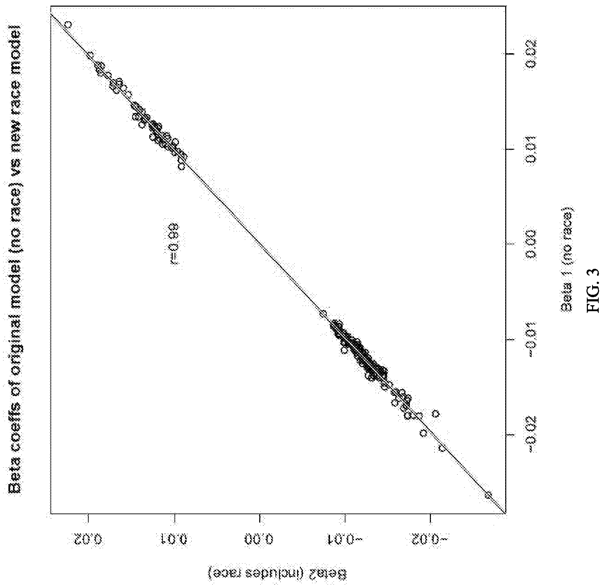

An inverse relationship between AOSI and DNAm was observed at 141 (73%) of these regions. These DMRs overlapped genes (87 were located within 10 kb of a gene) that were enriched for gene ontology biological processes of cellular movement, neurogenesis, and neuronal development categories (Table 3). Sensitivity analysis involving linear regression on log-transformed AOSI scores [ln(AOSI+1)] yielded consistent results with the bump hunting performed on the linear AOSI models described above - all 193 DMRs matched directionality with regression statistics and 144/193 regions (74.6%) had p <0.05. Similarly, when performing linear regression using a model of the highest quartile of AOSI score relative to the other three quartiles, the results were also consistent with the primary analyses - all 193 DMRs matched directionality with regression statistics and 142/193 regions (73.6%) had p<0.05. Results from bump hunting analyses that adjusted for principal components of race were not substantially different from those reported. Beta coefficients for DMRs before and after race adjustment were highly correlated (p=0.99, Figure 3).

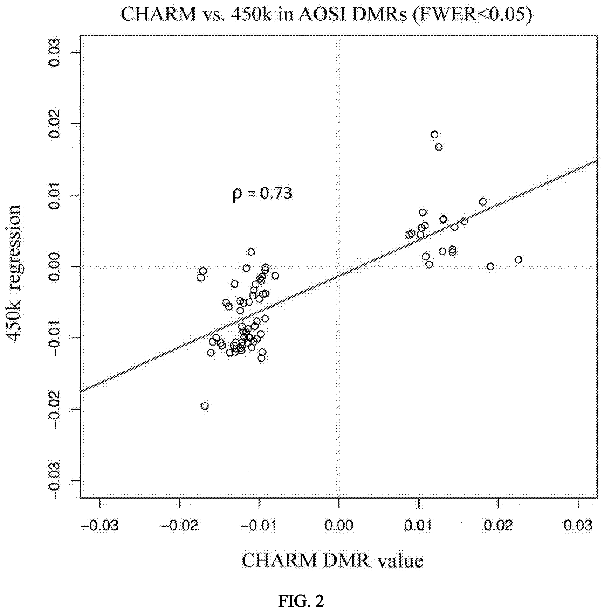

Among these 193 AOSI-associated DMRs, Illumina 450k™ probes located within 500bp of the region boundaries were extracted. This was possible for 75 (38.9%) CHARM-discovered DMRs (Table 7) represented by 244 probes on the 450K array. The direction of association between AOSI and DNAm was consistent with CHARM results for 74 (98.7%) of these 75 regions (Figure 2).

### AOSI-associated DMRs in postmortem brain

A subset of the AOSI-associated DMRs identified in semen also shows directionally consistent effects in postmortem human brain samples in a study comparing patients with ASD to unaffected controls. The AOSI DMRs were most consistent comparing cases and controls in the cerebellum, where 21 of 75 of AOSI-associated DMRs contained at least one *Illumina* 450K probe that was differentially methylated comparing autism to controls; 18 of these were directionally consistent with the sperm AOSI score association data (e.g. DNAm positively associated with both AOSI score in semen and in ASD brains). This cross-tissue consistency was not present in the cortical brain regions - in the frontal cortex, only 12 AOSI DMRs were significant, of which 3 were directionally consistent, and in the temporal cortex, only 10 AOSI DMRs were significant, of which 2 were directionally consistent. Surprisingly, 15 of 18 cerebellum-consistent AOSI DMRs were overlapping genes in the SNORD families (Table 8).

### FIGURE LEGENDS

Figures 1A-1D are graphical representations of methylation plots for the top 4 statistical DMRs identified using CHARM and 12-month score in embodiments of the present invention. (A) SNORD115-15, (B) SNORD115-11, (C) SNORD115-17, (D) SMYD3. Top panels show individual methylation levels at each probe by genomic position. Dotted vertical lines represent the boundaries of the DMR, and coloured lines represent the average methylation curve for samples grouped by quartiles of AOSI scores-the scores within each quartile are shown in the legend. Middle panel shows location of CpG dinucleotides (as black tick marks) and CpG density by genomic position (black curved line). Bottom panel shows location (boxes) and direction (+ or -) of refseq-annotated genes. Inset scatterplot reflects linear regression of average methylation across all probes within DMR per sample by AOSI 12-month score.

Figure 2 is a graphical representation depicting validation of CHARM AOSI DMR values using 450k data from overlapping samples (n=30). The CHARM DMR value corresponds to the smoothed effect estimate at each probe, plotted against the single-site regression coefficients from 450 K data for each corresponding DMR. Spearman rank correlation coefficient is also included.

Figure 3 is a graphical representation showing the relationship between linear regression beta coefficients of the original bump hunting model (Beta1) and the model including principal components of race from ancestry analysis of GWAS SNP data (Beta2). Spearman rank correlation coefficient also included.

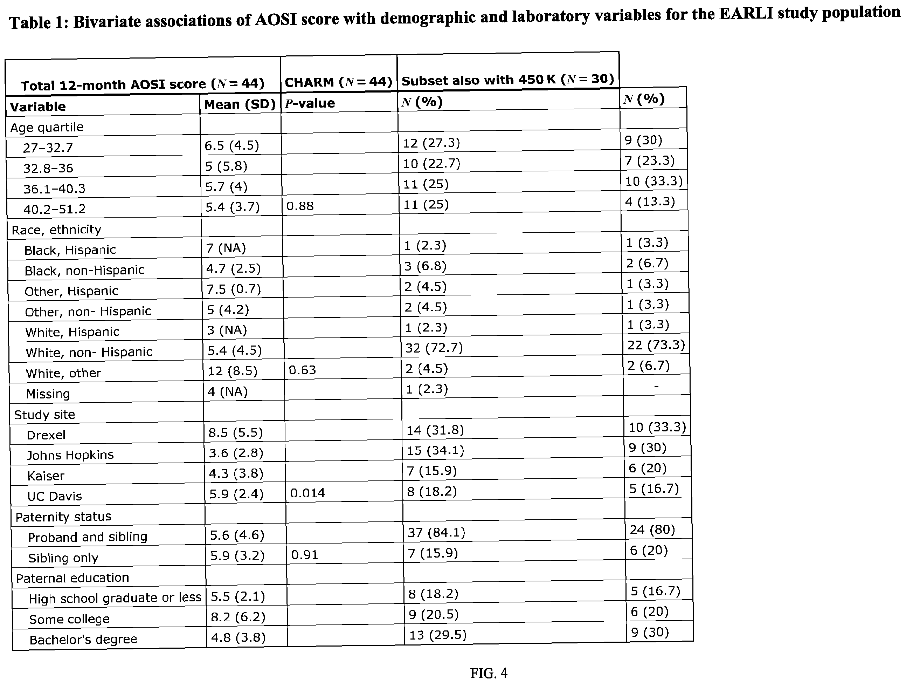

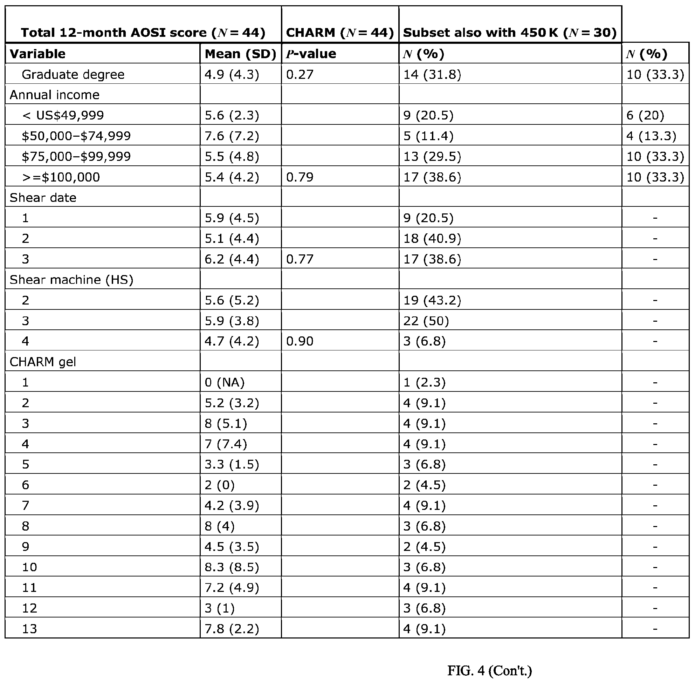

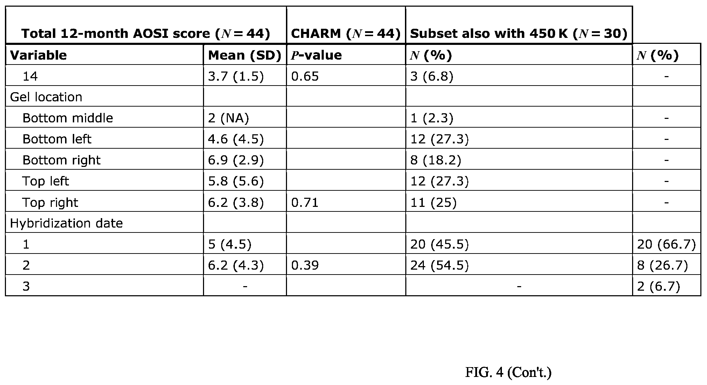

Figure 4 sets forth Table 1 relating to bivariate associations of AOSI score with demographic and laboratory variables for the EARLI study population.

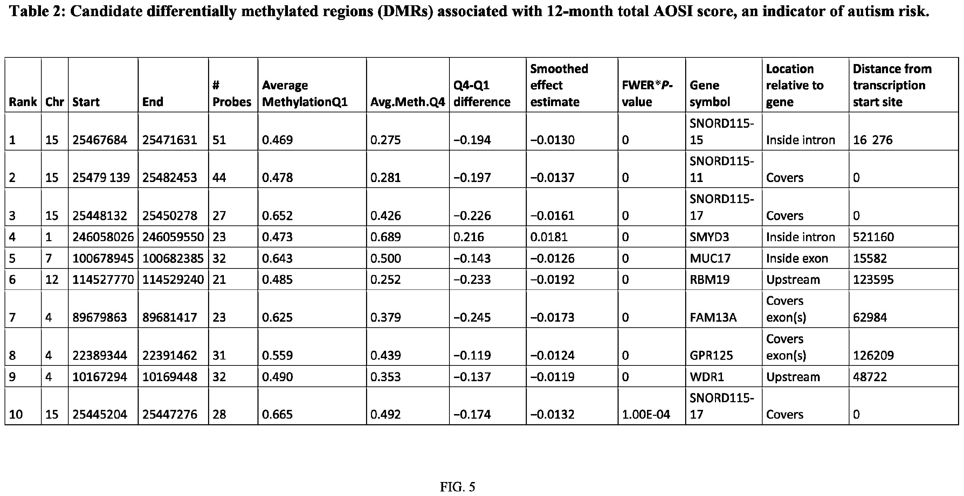

Figure 5 sets forth Table 2 relating to candidate differentially methylated regions (DMRs) associated with 12-month total AOSI score, an indicator of autism risk.

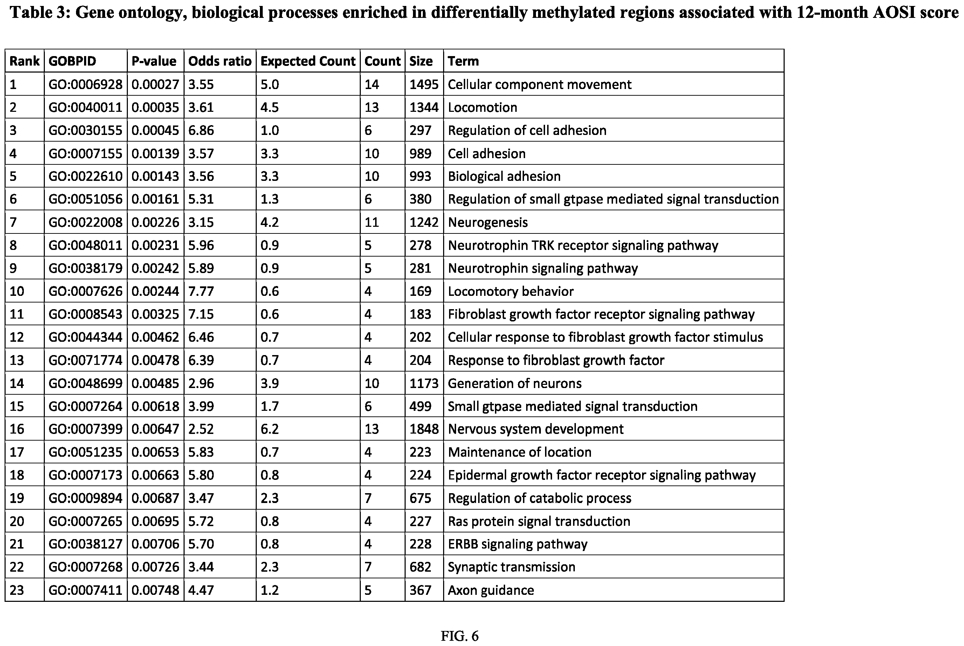

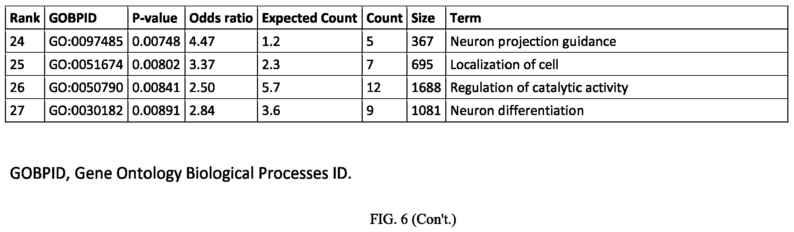

Figure 6 sets forth Table 3 relating to gene ontology, biological processes enriched in differentially methylated regions associated with 12-month AOSI score.

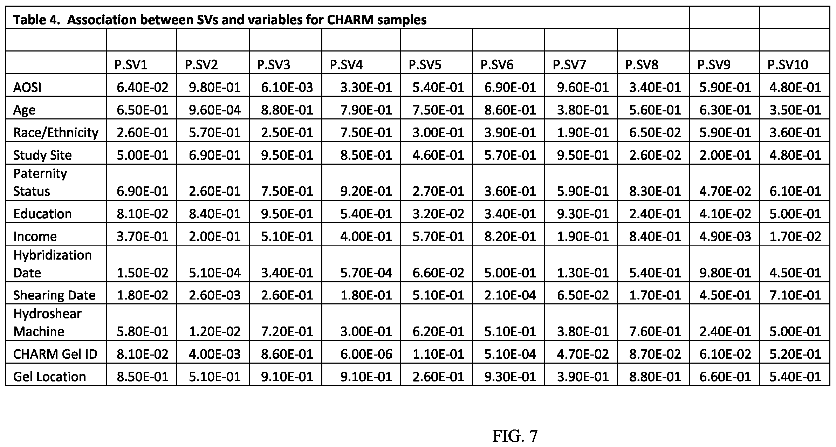

Figure 7 sets forth Table 4 relating to association between SVs and variables for CHARM samples.

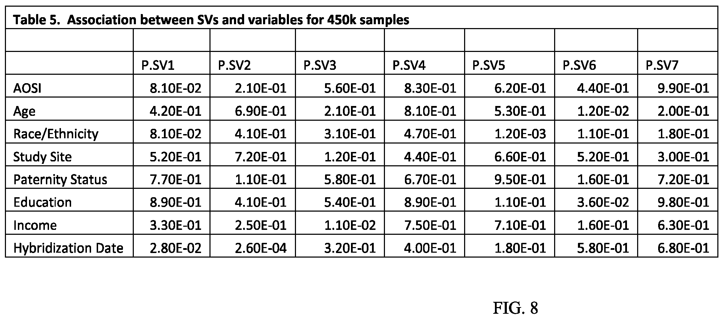

Figure 8 sets forth Table 5 relating to association between SVs and variables for 450k samples.

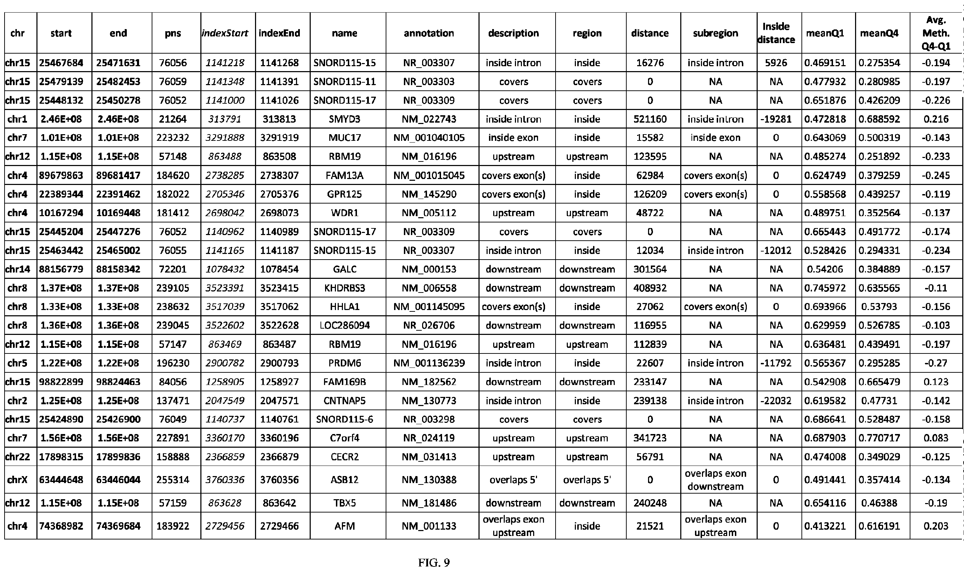

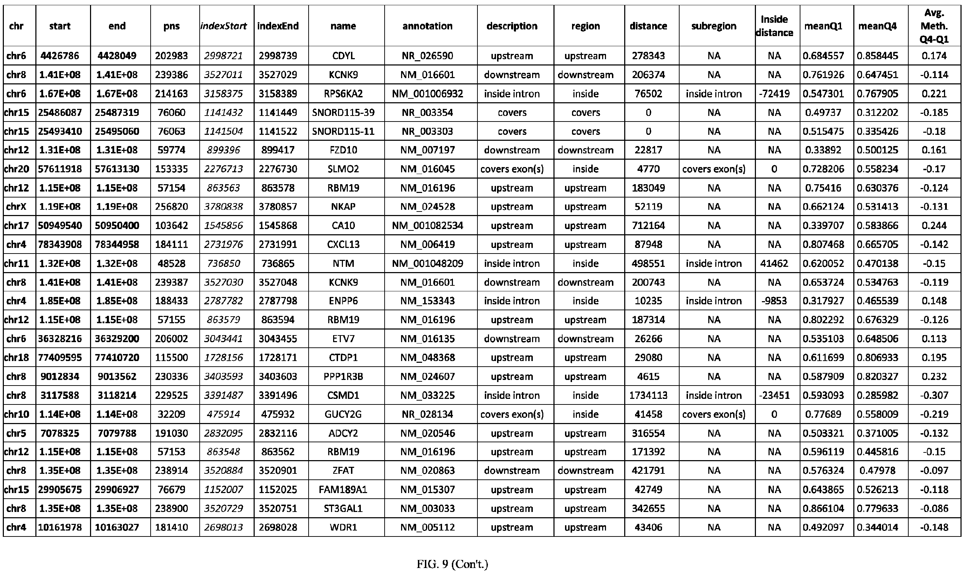

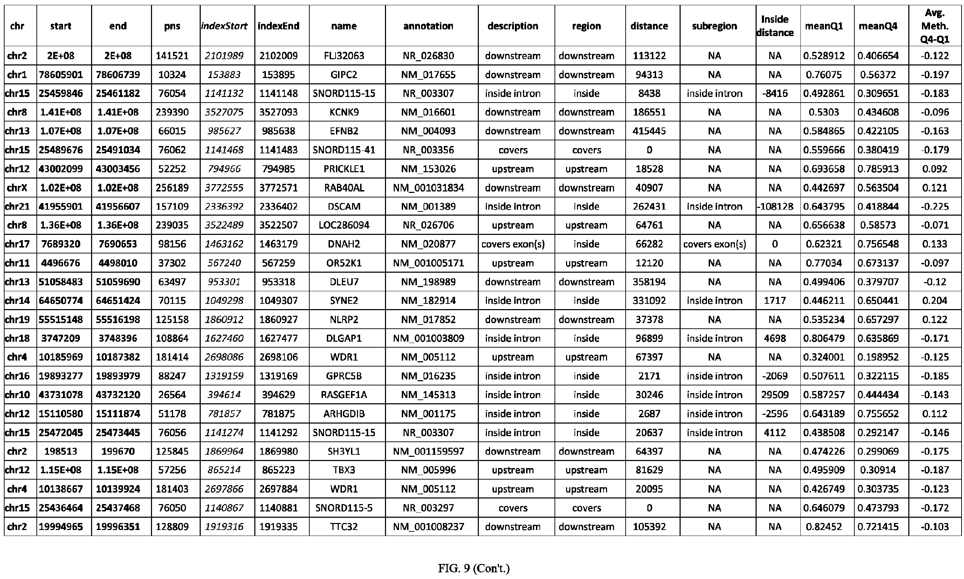

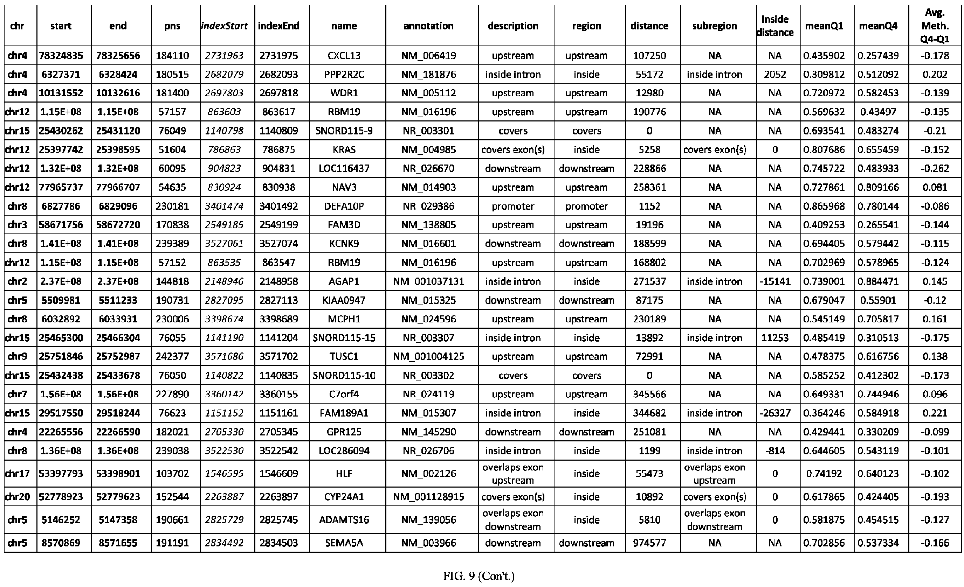

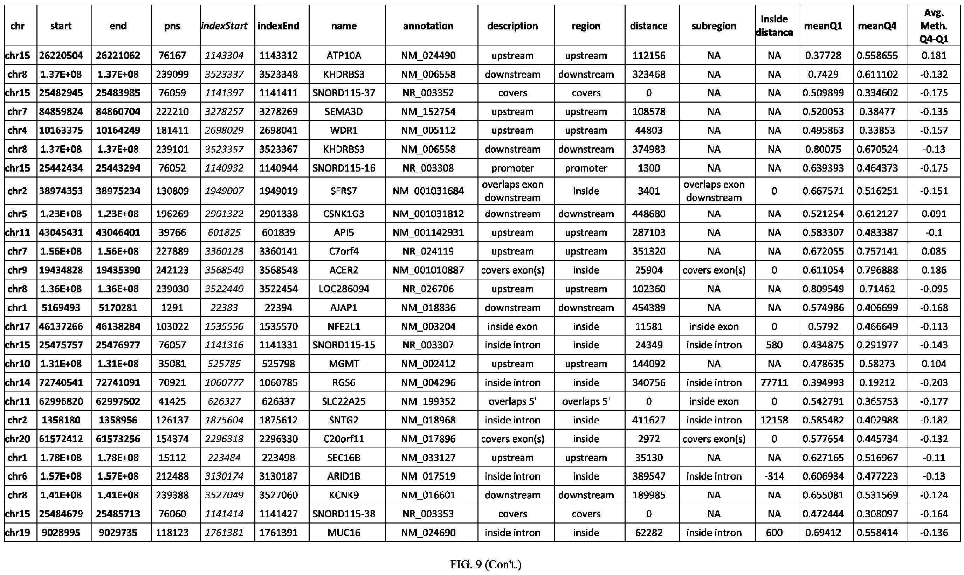

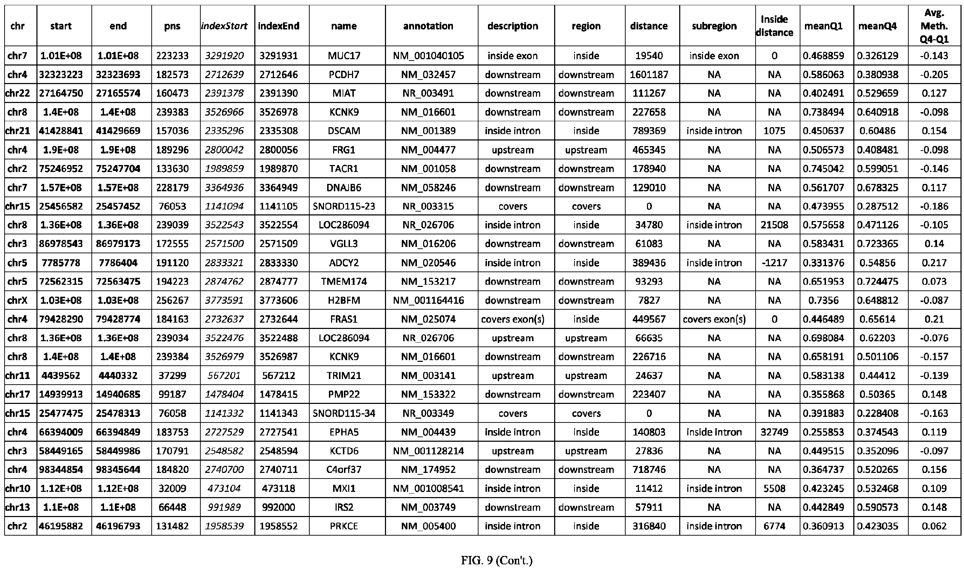

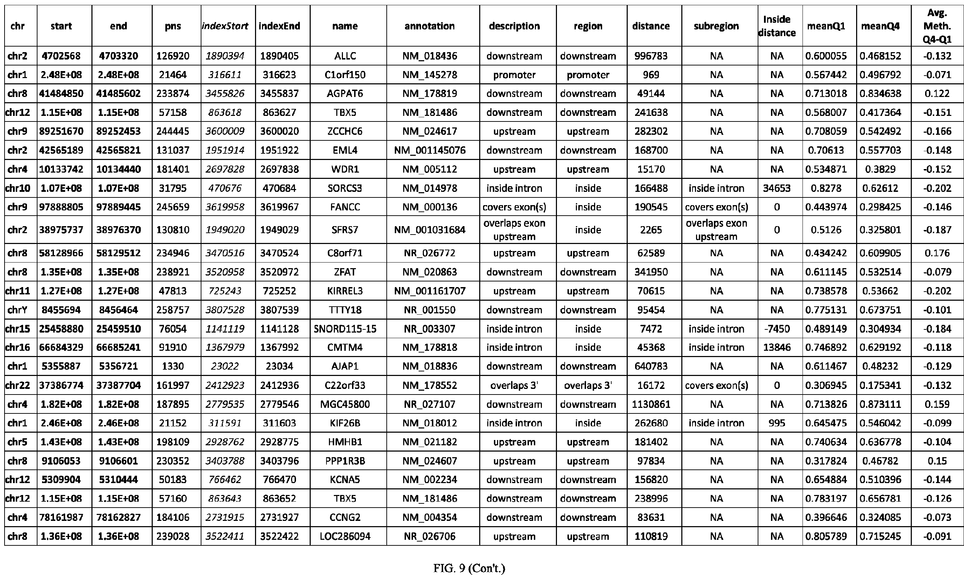

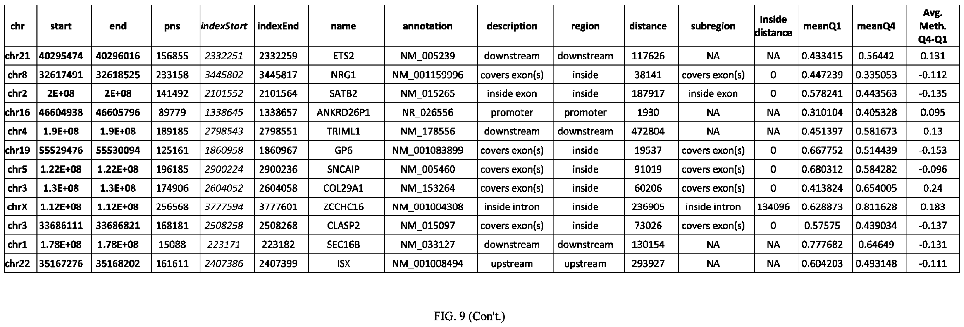

Figure 9 sets forth Table 6 relating to the top 193 DMRs associated with autism as determined by an embodiment of the invention.

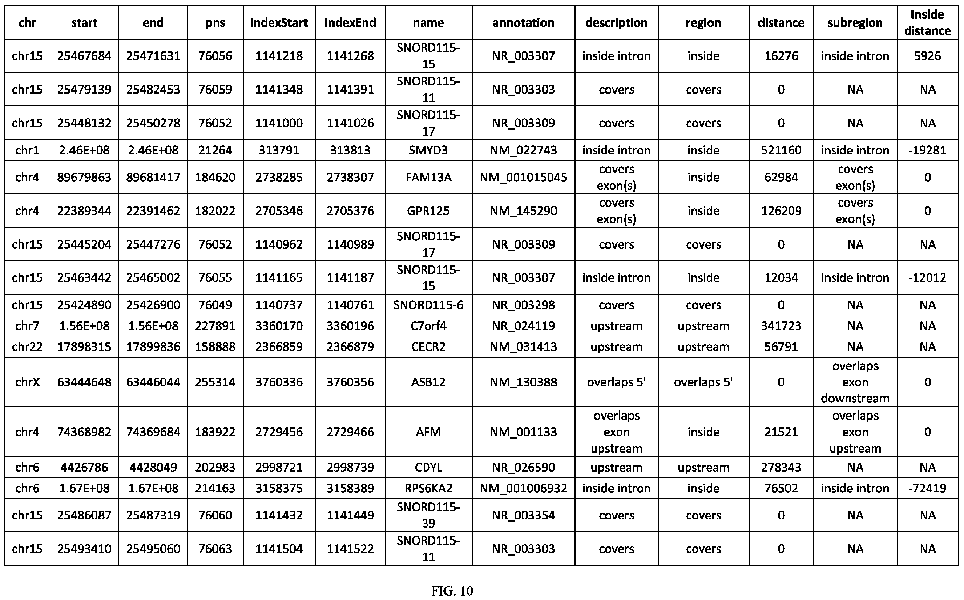

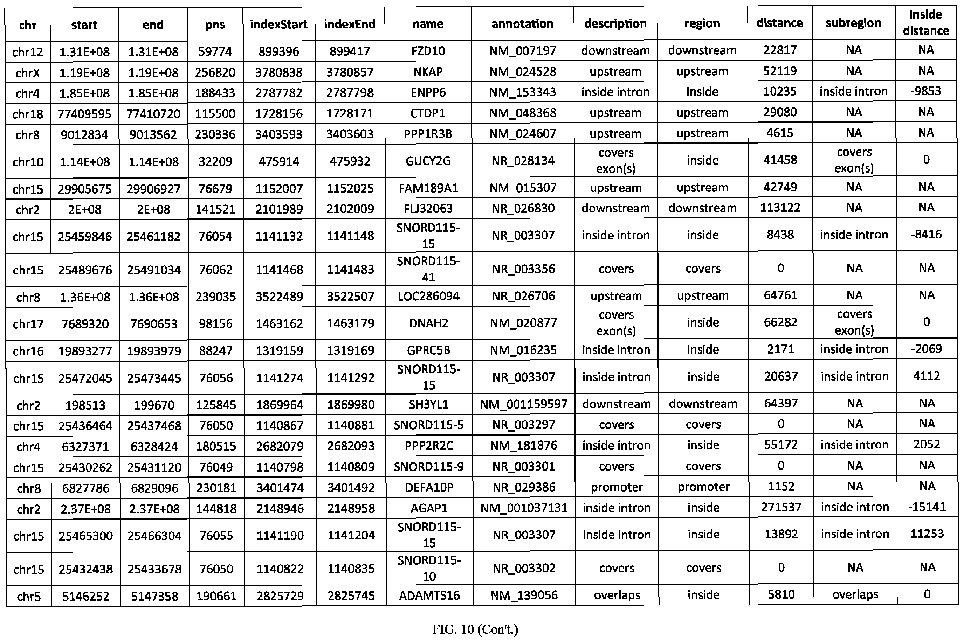

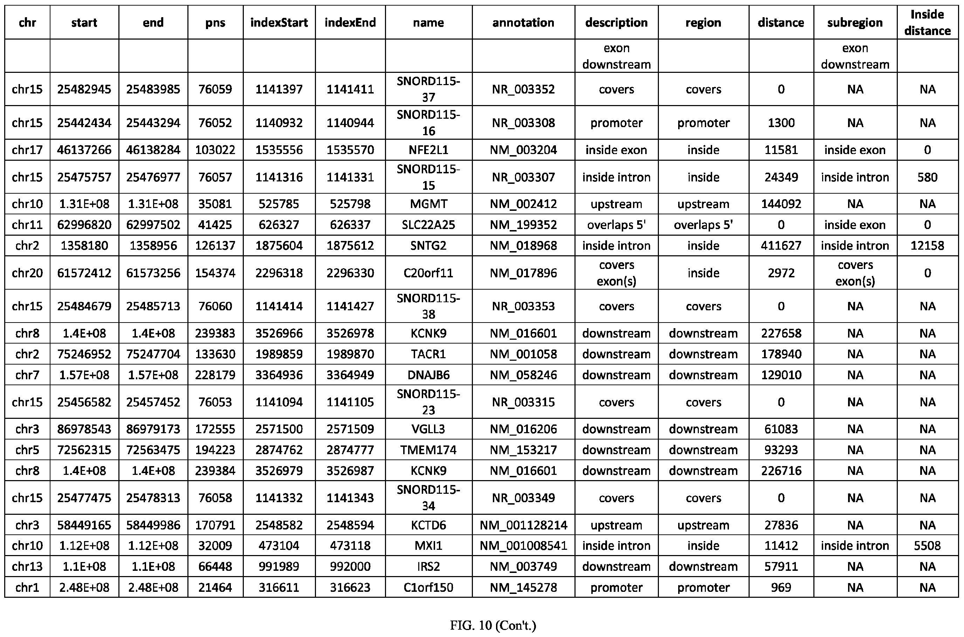

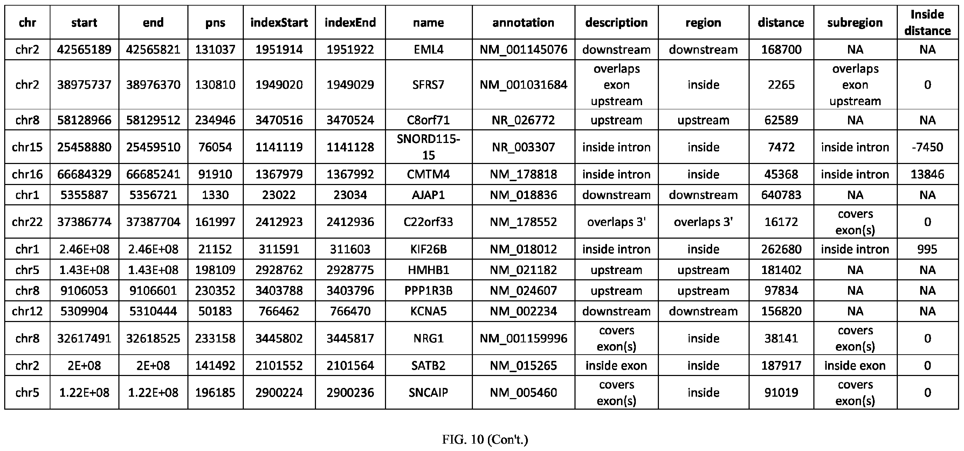

Figure 10 sets forth Table 7 relating to validation of 450k probes.

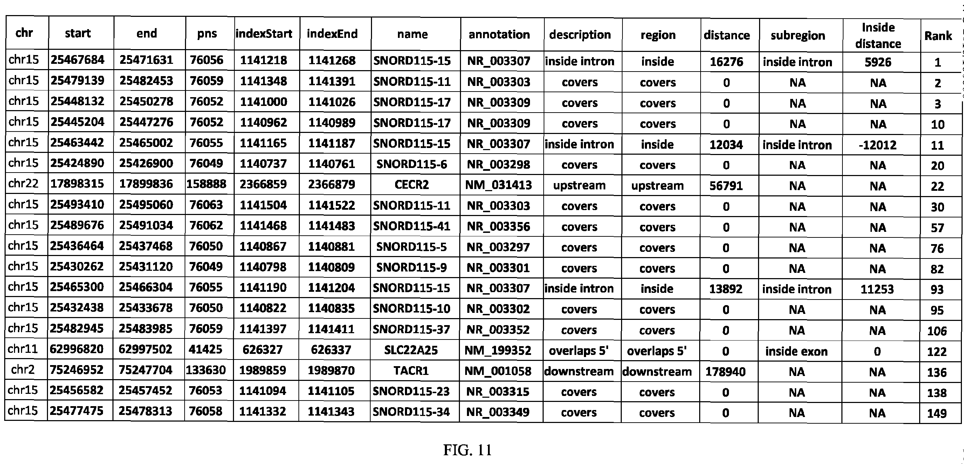

Figure 11 sets forth Table 8 relating to overlap of AOSI DMRs.

### DISCUSSION

In summary, a strong relationship between DNA methylation in sperm from fathers and 12-month ASD-related phenotype in infant siblings within an enriched autism risk birth cohort was observed. The degree of methylation change was quite substantial compared to recent reports for other epigenome-wide association studies (EWAS): 6.2 - 30.7% difference between top and bottom quartiles of AOSI score among the 193 DMRs with FWER < 0.05. These AOSI-associated DMRs showed enrichment for genes involved in neurogenesis and more general neuronal development.

12-month infant developmental assessment as a measure of early ASD-related phenotype was focused on. Total scores on the AOSI administered to high-risk infants at 12-months has shown high inter-rater reliability (0.93), test-retest reliability (0.61) and correlation with ASD diagnosis at age three. Because of the unusual nature of this prospective high-risk cohort, direct replication is difficult. However, an independent data set of autism and control brains showed overlap with the AOSI-associated DMRs, with 18 of 21 genes directionally consistent in methylation values.

How might these DMRs contribute to ASD? The sperm contributes half of the genome to somatic cells, and after germline reprogramming (except for generating germ cells in the next generation), this would likely persist into brain development. The fact that these genes were enriched for neurological function is consistent with this idea. Remarkably, 15 members of the small nucleolar RNA, C/D Box (SNORD-115) genes were in this overlapping set. This gene cluster lies within the Prader-Willi critical region of chromosome 15 and are considered candidate mechanistic targets for the disorder, a canonical imprinted Mendelian disorder with autism features. Gene dosage alterations in this region are also associated with ASD. The data reported here suggest that paternal epigenetic changes of this gene cluster confer risk of ASD among offspring, at least among those with an older affected sibling. Molecularly, aberrant imprinting, as seen in Prader-Willi syndrome, would not explain these results as the paternal allele is normally expressed.

The association with the cerebellum is intriguing. A case report of twins discordant for ASD showed cerebellar anatomical changes in the affected twin, and some neuro-imaging studies have shown changes in cerebellum in autism, or autistic features in children with cerebellar defects.

One potential limitation of the study is that DNA was extracted from semen rather than purified sperm. However DNA methylation has been shown to be highly uniform regardless the quality of sperm subpopulations. Thus, there was no adjustment for sperm quality or subpopulations. Also, there has been recent increased awareness of possible bias in enrichment statistics when gene size is not considered. No difference between gene size (p=0.055), coding length (p=0.79), and GC content (p=0.1) comparing DMRs with FWER < 0.05 (N=193) to those with FWER > 0.05 (N=2412) was found. Further, the average gene length was actually shorter for AOSI DMRs in our results (144kb vs 178kb). Additionally, the CHARM design features sets of probe groups, each with very evenly spaced probes (56-90bp), which was designed solely to target regions of at least moderate CpG density in the genome in an annotation-agnostic manner. Note this is different than the more promoter-focused designs, where probe density has been shown to bias gene set analysis. The CHARM method is less quantitative at individual loci due to the use of a restriction enzyme and how signal is measured. However, it was previously shown that it is more powerful for identifying relative changes in DNAm at the region level.

While the prevalence of autism spectrum disorder (ASD) has been increasing in recent years, even the current estimate of 1-2% (2) makes study of a general population prospective pregnancy cohort not feasible for ASD. For diseases such as ASD that demonstrate familial aggregation, disease-specific enriched-familial risk cohort studies are a commonly employed design. There is a long history of enriched-risk (family-based) cohort studies in epidemiology to examine risk factors for rare diseases. These studies are not designed to be generalizable to the general population, though targeted results from an enriched risk cohort can be replicated in a representative population to test generalizability. Nevertheless, in our study, fathers of second children with high- and low- AOSI scores are from the same underlying EARLI enriched-risk population, and thus effect estimates of association are not distorted due to selection bias, but in fact internally valid.

In summary, relatively large inter-individual differences in paternal sperm DNAm were identified that associate with later 12-month ASD-related phenotype in their biological offspring, among a sample of enriched-risk ASD infant siblings. Many of these regions of paternal sperm DNAm were also associated with ASD in cerebellum brain samples. Further, the genes implicated are enriched for neurodevelopment and include regions implicated in Prader-Willi syndrome. This unique set of biosamples is comparatively small, given the nature of the biosample type and required perinatal collection timing in an enriched risk cohort. Thus it is important to examine these findings in larger samples to confirm these associations and explore mechanistic implications.

## Claims

1. A method for determining risk of autism spectrum disorder (ASD) in an offspring subject comprising analyzing DNA methylation status in a sample containing sperm from a prospective paternal parent, wherein a methylation pattern that is different from the pattern found in a sample not associated with ASD is indicative of a risk of ASD in the offspring,
wherein determining a methylation status comprises determining the methylation at differentially methylated regions (DMRs) in the DNA, and
wherein the DMRs reside on SNORD115-15, SNORD115-11, SNORD115-17 and SMYD3.

2. A method for determining whether a subject has or is at risk of having autism spectrum disorder (ASD) comprising analyzing DNA methylation status in a DNA sample of the subject, wherein a methylation pattern that is different from the pattern found in a sample not associated with ASD is indicative of a risk of ASD in the subject,
wherein determining a methylation status comprises determining the methylation at differentially methylated regions (DMRs) in the DNA, and
wherein the DMRs reside on SNORD115-15, SNORD115-11, SNORD115-17 and SMYD3.

3. The method of claims 1 or 2, wherein the methylation status is performed by one or more techniques selected from the group consisting of a nucleic acid amplification, polymerase chain reaction (PCR), methylation specific PCR, bisulfite sequencing, capture bisulfite sequencing, whole genome bisulfite sequencing, pyrosequencing, single-strand conformation polymorphism (SSCP) analysis, restriction analysis, and microarray technology, including bead microarray technology.

4. The method of claim 1 or 2, wherein the method comprises performing comprehensive high-through array-based relative methylation (CHARM) analysis on a sample of labeled, digested genomic DNA.

5. The method of claim 2, wherein the risk is assessed as a score relative to low, moderate or high risk.

6. The method of claim 1 or 2, wherein the methylation status is hypomethylated or hypermethylated.

## Patentansprüche

1. Ein Verfahren zum Bestimmen des Risikos einer Autismusspektrumstörung (ASS) in einem Kindesindividuum, das das Analysieren des DNA-Methylierungszustands in einer Probe, die Sperma von einem potenziellen Vater enthält, beinhaltet, wobei ein Methylierungsmuster, das sich von dem Muster unterscheidet, welches in einer nicht mit ASS verknüpften Probe zu finden ist, ein Anzeichen für ein Risiko von ASS in dem Kind ist,
wobei das Bestimmen eines Methylierungszustands das Bestimmen der Methylierung an unterschiedlich methylierten Bereichen (DMB) in der DNA beinhaltet und
wobei sich die DMB auf SNORD115-15, SNORD115-11, SNORD115-17 und SMYD3 befinden.

2. Ein Verfahren zum Bestimmen, ob ein Individuum eine Autismusspektrumstörung (ASS) oder ein Risiko dafür aufweist, das das Analysieren des DNA-Methylierungszustands in einer DNA-Probe des Individuums beinhaltet, wobei ein Methylierungsmuster, das sich von dem Muster unterscheidet, welches in einer nicht mit ASS verknüpften Probe zu finden ist, ein Anzeichen für ein Risiko von ASS in dem Individuum ist,
wobei das Bestimmen eines Methylierungszustands das Bestimmen der Methylierung an unterschiedlich methylierten Bereichen (DMB) in der DNA beinhaltet und
wobei sich die DMB auf SNORD115-15, SNORD115-11, SNORD115-17 und SMYD3 befinden.

3. Verfahren gemäß Anspruch 1 oder 2, wobei der Methylierungszustand durch eine oder mehrere Techniken durchgeführt wird, die aus der Gruppe ausgewählt sind, welche aus einer Nukleinsäureamplifikation, Polymerase-Kettenreaktion (PCR), methylierungsspezifischen PCR, Bisulfitsequenzierung, Fangbisulfitsequenzierung, Bisulfitsequenzierung des gesamten Genoms, Pyrosequenzierung, Einzelstrang-Konformationspolymorphismus-Analyse (ESKP-Analyse), Restriktionsanalyse und Microarray-Technologie, einschließlich Perlen-Microarray-Technologie, besteht.

4. Verfahren gemäß Anspruch 1 oder 2, wobei das Verfahren das Durchführen einer Analyse relativer Methylierung auf Basis eines ausführlichen Arrays mit hohem Durchsatz (CHARM-Analyse) an einer Probe markierter, verdauter genomischer DNA beinhaltet.

5. Verfahren gemäß Anspruch 2, wobei das Risiko als ein Punktwert in Bezug auf ein niedriges, moderates oder hohes Risiko eingeschätzt wird.

6. Verfahren gemäß Anspruch 1 oder 2, wobei der Methylierungszustand hypomethyliert oder hypermethyliert ist.

## Revendications

1. Une méthode destinée à la détermination de risque de trouble du spectre de l'autisme (TSA) chez un sujet descendant comprenant l'analyse de l'état de méthylation de l'ADN dans un échantillon contenant du sperme d'un futur parent paternel, dans laquelle un motif de méthylation qui est différent du motif trouvé dans un échantillon non associé au TSA est indicatif d'un risque de TSA chez le descendant,
dans laquelle la détermination d'un état de méthylation comprend la détermination de la méthylation au niveau de régions méthylées différentiellement (DMR) dans l'ADN, et dans laquelle les DMR résident sur SNORD115-15, SNORD115-11, SNORD115-17 et SMYD3.

2. Une méthode destinée à la détermination quant à savoir si un sujet souffre ou court le risque de souffrir de trouble du spectre de l'autisme (TSA) comprenant l'analyse de l'état de méthylation d'ADN dans un échantillon d'ADN du sujet, dans laquelle un motif de méthylation qui est différent du motif trouvé dans un échantillon non associé au TSA est indicatif d'un risque de TSA chez le sujet,
dans laquelle la détermination d'un état de méthylation comprend la détermination de la méthylation au niveau de régions méthylées différentiellement (DMR) dans l'ADN, et dans laquelle les DMR résident sur SNORD115-15, SNORD115-11, SNORD115-17 et SMYD3.

3. La méthode des revendications 1 ou 2, dans laquelle l'état de méthylation est réalisé au moyen d'une ou de plusieurs techniques sélectionnées dans le groupe constitué d'une amplification d'acides nucléiques, d'une réaction en chaîne par polymérase (PCR), d'une PCR spécifique à la méthylation, d'un séquençage au bisulfite, d'un séquençage au bisulfite de capture, d'un séquençage au bisulfite du génome entier, d'un pyroséquençage, d'une analyse de polymorphisme de conformation des simples brins (SSCP), d'une analyse de restriction, et d'une technologie des microréseaux, y compris la technologie des microréseaux à billes.

4. La méthode de la revendication 1 ou de la revendication 2, la méthode comprenant la réalisation d'une analyse de méthylation relative basée sur réseaux à débit d'alimentation élevé (CHARM) sur un échantillon d'ADN génomique marqué, digéré.

5. La méthode de la revendication 2, dans laquelle le risque est évalué comme un score relativement à un risque faible, modéré ou élevé.

6. La méthode de la revendication 1 ou de la revendication 2, dans laquelle l'état de méthylation est hypométhylé ou hyperméthylé.
